(19) Europäisches Patentamt
European Patent Office
Office européen des brevets

(11) **EP 3 882 785 A1**

(12) **EUROPEAN PATENT APPLICATION**

(43) Date of publication:
**22.09.2021 Bulletin 2021/38**

(51) Int Cl.:
**G06F 16/335** (2019.01)   **G06F 40/00** (2020.01)
**G16B 45/00** (2019.01)

(21) Application number: **21152950.8**

(22) Date of filing: **22.01.2021**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.03.2020   JP 2020045980**

(71) Applicant: **Hitachi, Ltd.
Tokyo 100-8280 (JP)**

(72) Inventor: **IMAICHI, Osamu
Tokyo, 100-8280 (JP)**

(74) Representative: **MERH-IP Matias Erny Reichl
Hoffmann
Patentanwälte PartG mbB
Paul-Heyse-Strasse 29
80336 München (DE)**

(54) **DOCUMENT SEARCH SYSTEM AND METHOD**

(57)   Provided is a document search technique with which a user can efficiently find useful documents for the user. A system extracts one or more topic words from a set of seed documents of one or more seed documents, and creates a useful document model which is a model including the one or more topic words and a weight of each of the one or more topic words. A seed document is a document which may be a useful document. The system extracts one or more documents matching a search condition from a document search range including one or more documents according to a search request in which the search condition is specified. The system determines, for each of the one or more extracted documents, a document score of the document based on the above-described useful document model, and outputs a search result on descending order of document scores of the one or more extracted documents.

*FIG. 1*

**Description**

BACKGROUND OF THE INVENTION

1. Field of the Invention

The present invention generally relates to a document search technique.

2. Description of the Related Art

With spread of computers and the Internet, digitization of documents is progressing rapidly. For example, there is a life science system document database in which about 30 million documents are searched targets and more than 1 million documents are increasing every year. A user of the life science system finds useful documents which contribute to solving his research problems from the document database of such a large number of documents, and uses these useful documents for research and development.

A typical technique for searching the document database for a document includes a keyword search technique. In the keyword search, a search may be executed by combining a plurality of keywords . When no useful document is found, trial and error such as adding or deleting a keyword is repeated.

A technique different from the keyword search includes a similar document search technique. JP-A-2000-155758 (Patent literature 1) discloses an example of the similar document search technique.

In order to search for useful documents with a general keyword search technique, it is often necessary to combine the keywords by trial and error, which is not efficient. In addition, with the keywords selected by trial and error, a large number of documents may be hit and search omissions may occur.

For example, when searching for a metabolic reaction related to material production (production of a target compound) by a combination of a name (for example, pyruvate) of a substrate constituting the metabolic reaction, a name (for example, acetolactate synthase) of an enzyme, and a name (for example, 2-acetolactate) of a product, the number of hits is small and useful documents cannot be sufficiently obtained. Therefore, a method of searching by a name (for example, genes expressing acetolactate synthase include alsS, brnP, budB, ilvB, ilvB1, ilvB2, ilvG, ilvH, ilvI, ilvK, ilvM, ilvN, ilvX, and ilvY) of a gene expressing the enzyme is considered. However, this method assumes that the number of hits increases as more documents (documents as noise) which do not correspond to the useful documents are included. In order to obtain the useful documents related to the material production from search results under the name of the gene, it is considered to narrow down the search results with a keyword (for example, production, metabolic, engineering, biosynthesis, pathway) related to the material production. However, it is difficult to create a set of keywords for exhaustively searching for the useful documents for the material production without omission.

On the other hand, in order to search for the useful documents with the similar document search, a document matching a search request of the user needs to be provided as a search input. However, each time the search request of the user changes, it is necessary to search for the document serving as the search input, which is not efficient. Further, a search result in which a feature of the document serving as the search input is excessively reflected may be obtained, and accordingly a deviation may occur in the obtained search result. In other words, even when the document serving as the search input is an example of the useful documents, the hit documents may not necessarily correspond to the useful documents.

Another method is to create a discriminative model using a machine learning algorithm with useful documents and non-useful documents as correct data, and classify search results into the useful documents and the non-useful documents using the created discriminative model. However, in order to accurately classify with the machine learning algorithm, it is necessary to create a large amount of correct data, which is considered to be low in convenience.

The above problems can also be found in document searches other than the document search for the metabolic reaction.

SUMMARY OF THE INVENTION

In view of the above situation, an object of the invention is to provide a document search technique with which a user can efficiently find useful documents for the user.

A system extracts one or more topic words from a set of seed documents of one or more seed documents, and creates a useful document model which is a model including the one or more topic words and a weight of each of the one or more topic words. A seed document is a document which may be a useful document. The system extracts one or more documents matching a search condition from a document search range including one or more documents according to a search request in which the search condition is specified. The system determines, for each of the one or more extracted documents, a document score of the document based on the above-described useful document model, and outputs a search result on descending order of document scores of the one or more extracted documents.

According to the invention, the document score is determined for each document as the search result using the useful document model including the topic word (document set which may be a useful document) of the set of seed documents and the weight, and the search result is provided on descending order of the document scores. Accordingly, the user can efficiently find the useful documents for the user. Technical problems, configurations and effects other than those described above will be clarified by the following description of the embodiments.

BRIEF DESCRIPTION OF THE DRAWINGS

FIG. 1 shows a configuration example of a document search system according to a first embodiment.
FIG. 2 shows a configuration example of a topic word extraction unit.
FIG. 3 shows a configuration example of a document score giving unit.
FIG. 4 shows an example of a search request input screen on a search client.
FIG. 5 shows an example of a search result screen on the search client.
FIG. 6 shows an example of a seed document setting screen on a seed document setting client.
FIG. 7 is a sequence diagram of processing of registering a set of seed documents.
FIG. 8 is a sequence diagram of processing of searching for a useful document.
FIG. 9 shows an outline of a second embodiment.
FIG. 10 shows an outline of a third embodiment.
FIG. 11 shows an outline of a fourth embodiment.

DESCRIPTION OF THE PREFERRED EMBODIMENTS

Hereinafter, embodiments of the invention will be described with reference to the drawings. The embodiments of the invention are not limited to embodiments to be described below, and various modifications can be made within the scope of the technical idea thereof.

In the following description, a "communication interface device" may be one or more communication interface devices. The one or more communication interface devices may be one or more communication interface devices of the same type (for example, one or more network interface cards (NICs)), or be two or more communication interface devices of different types (for example, an NIC and a host bus adapter (HBA)).

In the following description, a "memory" may be one or more memory devices which is an example of one or more storage devices, and typically may be a main storage device. At least one memory device in the memory may be a volatile memory device or a non-volatile memory device.

In the following description, a "persistent storage device" may be one or more persistent storage devices which are an example of one or more storage devices. Typically, the persistent storage device may be a non-volatile storage device (for example, an auxiliary storage device), and specifically, may be a hard disk drive (HDD), a solid state drive (SSD), a non-volatile memory express (NVMe) drive, or a storage class memory (SCM).

In the following description, a "storage device" may be either the memory or the persistent storage device.

In the following description, a "processor" may be one or more processor devices. Typically, at least one processor device may be a microprocessor device such as a central processing unit (CPU), and may be another type of processor device such as a graphics processing unit (GPU) . The at least one processor device may be single-core or a multi-core. The at least one processor device may be a processor core. The at least one processor device may be a processor device in a broad sense such as a circuit (for example, a field-programmable gate array (FPGA), a complex programmable logic device (CPLD), and an application specific integrated circuit (ASIC)) which is an aggregate of gate arrays according to a hardware description language which executes a part or all of processing.

In the following description, an expression such as "xxx table" may be used to describe information which is obtained as an output for an input. The information may be data of any structure (for example, may be structured data or unstructured data), or may be a learning model such as a neural network which generates an output for an input, a genetic algorithm, and a random forest. Therefore, the "xxx table" can be referred to as "xxx information". In the following description, a configuration of each table is an example. One table may be divided into two or more tables, and all or a part of the two or more tables may be one table.

In the following description, an expression of "xxx unit" may be used to describe a function. The function may be implemented by a processor executing one or more computer programs, by one or more hardware circuits (for example, an FPGA or an ASIC), or by a combination of the above implementation methods. When the function is implemented by the processor executing the program, the function may be at least a part of the processor since predetermined processing is executed by appropriately using a storage device and/or an interface device. Processing described using the function as a subject may be processing executed by a processor or by a device including the processor. The program may be installed from a program source. The program source may be, for example, a program distribution computer or a recording medium (for example, a non-transitory recording medium) which can be read by a computer. A description for each function is an example. A plurality of functions may be combined into one function, and one function may be divided into a plurality of functions.

In the following description, a "document search system" may be a system constituted by one or more physical computers, or may be a system implemented based on a plurality of types of computational resources possessed by the one or more physical computers. For example, when a computer includes a display device and displays information on its own display device, the computer may be a document search system. For example, when a first computer (for example, a server) transmits display information to a second remote computer (display computer (for example, a client) and the display computer displays the information (when the first computer displays the information on the second computer), at least the first computer of the first computer and the second computer may be the document search system. The document search system "displays information" may mean displaying the information on a display device provided in a computer in the document search system, or may mean that the document search system transmits the information to a remote computer which displays the information (in the latter case, the information is displayed by the remote computer).

In the following description, a common reference numeral of reference numerals may be used when elements of the same type are described without distinction, and the reference numerals may be used when the elements of the same type are distinguished.

In the following description, "document" is a document which has been digitized.

[First Embodiment]

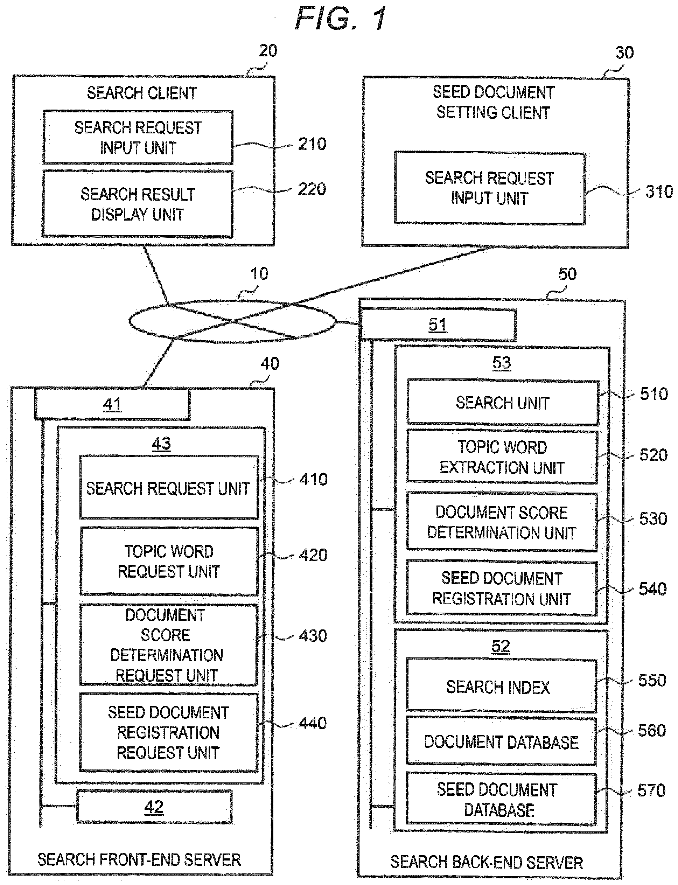

FIG. 1 shows a configuration example of a document search system according to a first embodiment.

This system includes a search client 20 used to input a search request by a user and display a search result, a seed document setting client 30 used to set a seed document for calculating a document score, a search back-end server 50 used to search for a document from a document database 560, extract a topic word from the document database 560, give a document score to the document, and register the seed document, and a search front-end server 40 which mediates among the search client 20, the seed document setting client 30, and the search back-end server 50. The search client 20, the seed document setting client 30, the search front-end server 40, and the search back-end server 50 are connected to a communication network 10.

In the example in FIG. 1, the search client 20, the seed document setting client 30, the search front-end server 40, and the search back-end server 50 are connected to the communication network 10, and a part or all of the search client 20, the seed document setting client 30, the search front-end server 40, and the search back-end server 50 may be configured on the same computer. Further, for example, there may be no search front-end server 40, and the search back-end server 50 may serve as a search server and receive the search request from the search client 20 and the seed document setting client 30.

The search client 20 is, for example, a computer such as a personal computer or a smartphone. The search client 20 includes a search request input unit 210 which receives the search request from the user and transmits the search request to the search front-end server 40, and a search result display unit 220 which displays a search result from the search front-end server 40. At least one of the search request input unit 210 and the search result display unit 220 may be implemented by executing a dedicated program (for example, a dedicated application program) by the search client 20, or may be implemented by executing a general-purpose program (general-purpose Web browser) by the search client 20.

The seed document setting client 30 is, for example, a computer such as a personal computer or a smartphone. The seed document setting client 30 includes a search request input unit 310 which receives the search request from the user and transmits the input search request to the search front-end server 40. The search request input unit 310 may be implemented by executing a dedicated program (for example, a dedicated application program) by the seed document setting client 30, or may be implemented by executing a general-purpose program (general-purpose Web browser) by the seed document setting client 30.

The search front-end server 40 includes, for example, a communication interface device 41 connected to the communication network 10, a storage device 42, and a processor 43 connected to the communication interface device 41 and the storage device 42. When the processor 43 executes one or more programs stored in the storage device 42, a search request unit 410, a topic word request unit 420, a document score determination request unit 430, and a seed document registration request unit 440 are implemented. The search request unit 410 receives the search requests transmitted from the search request input units 210 and 310, and transmits the search requests to the search back-end server 50. The topic word request unit 420 transmits a request for acquiring a topic word from a seed document database 570 provided in the search back-end server 50 to a topic word extraction unit 520 provided in the search back-end server 50. The document score determination request unit 430 transmits a request for determining (calculating) a document score for each document constituting a document set searched by a search unit 510 provided in the search back-end server 50 to a document score determination unit 530 provided in the search back-end server 50. The seed document registration request unit 440 transmits a request for registering a set of seed documents created by a seed document setting procedure to be described later in the seed document database 570 provided in the search back-end server 50 to a seed document registration unit 540 provided in the search back-end server 50.

The search back-end server 50 includes, for example, a communication interface device 51 connected to the communication network 10, a storage device 52, and a processor 53 connected to the communication interface device 51 and the storage device 52. The storage device 52 stores a search index 550, the document database 560, and the seed document database 570. When the processor 53 executes one or more programs stored in the storage device 52, the search unit 510, the topic word extraction unit 520, the document score determination unit 530, and the seed document registration unit 540 are implemented. The search unit 510 searches the document database 560 using the search index 550 in response to the request from the search request unit 410. The topic word extraction unit 520 extracts the topic word from the document set provided in the document database 560 and the seed document database 570 in response to the request from the topic word request unit 420. The document score determination unit 530 determines the document score for each document constituting the document set as a search result obtained by the search unit 510 in response to the request from the document score determination request unit 430. The seed document registration unit 540 registers the set of seed documents created by the seed document setting procedure to be described later in the seed document database 570 in response to the request from the seed document registration request unit 440.

The search unit 510 searches the document database 560 using the search index 550. The search here can be implemented by, for example, a known keyword search method. In this keyword search method, in order to improve efficiency of search processing, documents contained in the document database 560 are divided into words (for example, morphological analysis is executed on Japanese documents and stemming is executed on English documents), and the search index 550 which contains information indicating which word is included in which document is created in advance. When the search is executed, the search unit 510 can execute the search processing at a high speed by using the search index 550 created in advance. In the example in FIG. 1, the search unit 510 creates the search index 550 in advance for the document database 560 of the search back-end server 50 and uses the search index 550 for the search processing.

The document database 560 may be an example of a document store including one or more documents. The seed document database 570 may be an example of a seed document store including one or more seed documents. The "store" may be a set of documents or a logical storage space in which the set of documents is stored. The "store" may be a structured store or an unstructured store. Further, at least a part of the search index 550, the document database 560, and the seed document database 570 may be present in a storage external to the search back-end server 50.

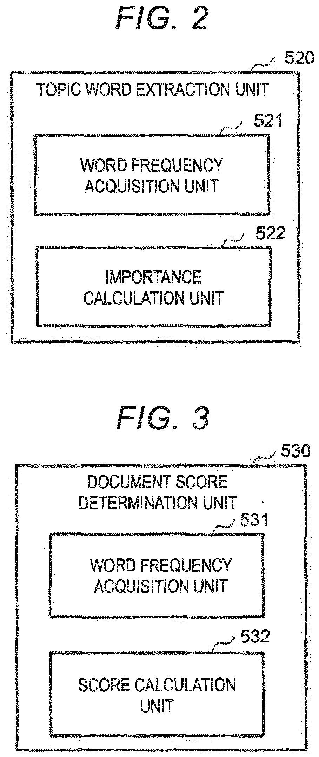

FIG. 2 shows a configuration example of the topic word extraction unit 520.

The topic word extraction unit 520 includes a word frequency acquisition unit 521 which acquires information indicating frequency of words contained in the documents of the document database 560, and an importance calculation unit 522 which calculates importance of the words using the acquired information (frequency information indicating the frequency of each word). The search index 550 is used in the same manner as the search unit 510 in order to implement fast extraction of topic words. That is, the topic word extraction unit 520 checks which word is included in which document with reference to the search index 550.

The extraction of the topic words is executed, for example, in the following procedure. First, the topic word extraction unit 520 receives the request transmitted from the topic word request unit 420 of the search front-end server 40. A document set is associated with the request. The word frequency acquisition unit 521 acquires frequency information of each word included in the document set. The importance calculation unit 522 calculates the importance of each word based on the acquired frequency information. A method of calculating the importance may be freely chosen. For example, the importance of the word may be calculated by a tf * idf method (for example, Equation 1 to be described later using the tf * idf method). The topic word extraction unit 520 returns words to the search front-end server 40 in descending order of importance as the topic words.

FIG. 3 shows a configuration example of the document score determination unit 530.

The document score determination unit 530 includes a word frequency acquisition unit 531 which acquires the frequency information of each word included in the documents obtained by the search unit 510, and a score calculation unit 532 which calculates a document score (importance of the document) using a topic word set obtained by the topic word extraction unit 520 and the frequency information of each word obtained by the word frequency acquisition unit 531. A method of calculating the document score may be freely chosen. For example, the document score may be calculated by the tf * idf method. The document score determination unit 530 returns documents to the search front-end server 40 in descending order of scores.

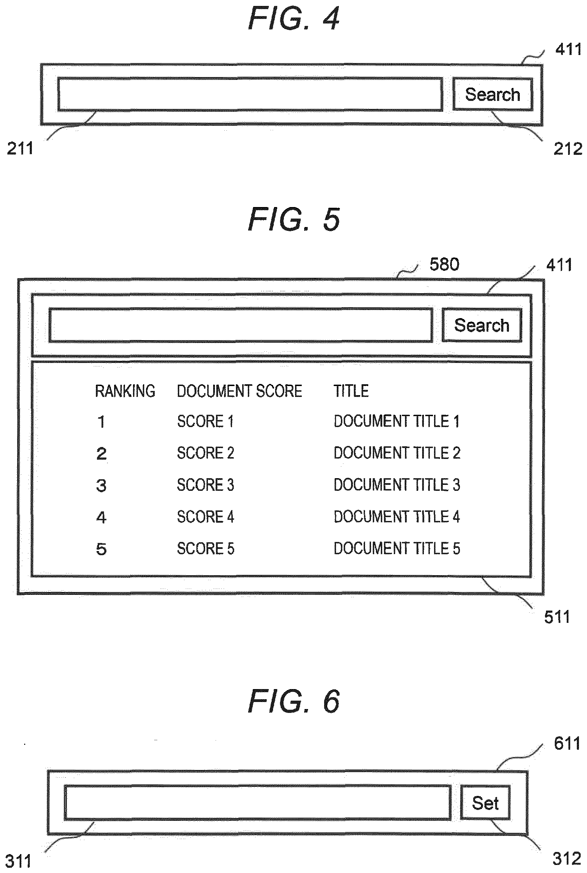

FIG. 4 shows an example of a search request input screen on the search client 20.

The search request input unit 210 displays a search request input screen 411. The search request input screen 411 includes, for example, a search input area 211 where a search input (for example, one or more keywords or search expressions thereof) is input, and a search instruction button 212 for instructing a search according to the search input in the search input area 211. The user inputs the search input to the search input area 211, and presses (for example, clicks) the search instruction button 212. Accordingly, the search client 20 is instructed to execute the search. The search request input unit 210 transmits the search request including the input search input to the search front-end server 40.

FIG. 5 shows an example of a search result screen on the search client 20.

A search result screen 580 is displayed by the search result display unit 220. The search result screen 580 includes, in addition to the search request input screen 411 shown in FIG. 4, for example, a document list screen 511 of documents found in the search according to the search input via the screen 411. The document list screen 511 includes a document ranking, a document score, and a document title obtained as search results. On the screen 511, for example, the document titles are arranged in descending order of document scores . A display format may be any format such as a tabular form or a list format. In the example of FIG. 5, the search request input screen 411 is displayed as a component of the search result screen 580 in order to perform re-search. However, the screen 411 may not necessarily be displayed on the search result screen 580.

FIG. 6 shows an example of a seed document setting screen on the seed document setting client 30.

The search request input unit 310 displays a seed document setting screen 611. The seed document setting screen 611 includes, for example, a search input area 311 where a search input (for example, one or more keywords or search expressions thereof) is input, and a seed document setting button 312 for instructing to register the documents found according to the search input in the search input area 311 as seed documents. The user inputs the search input to the search input area 311, and presses (for example, clicks) the seed document setting button 312. Accordingly, the seed document setting client 30 is instructed to execute seed document setting. The search request input unit 310 transmits the search request including the input search input to the search front-end server 40.

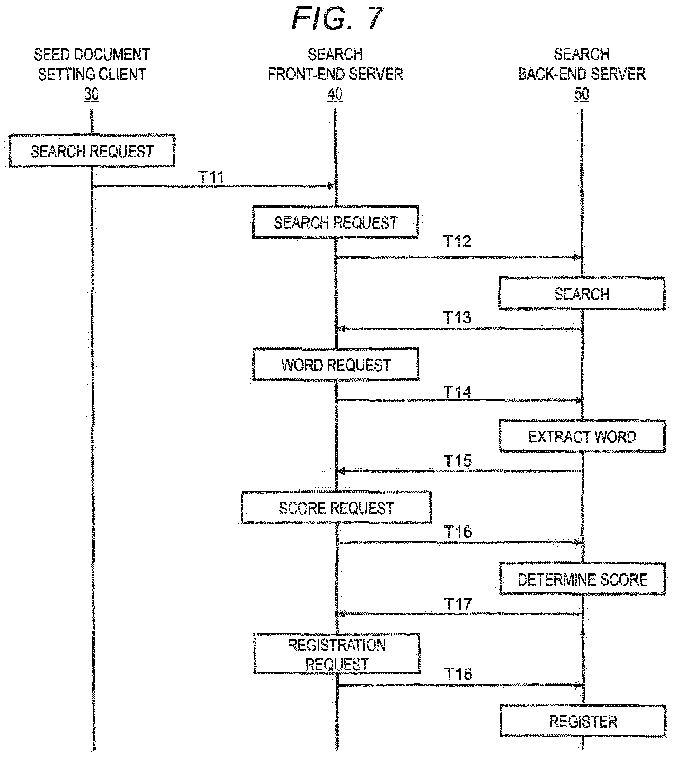

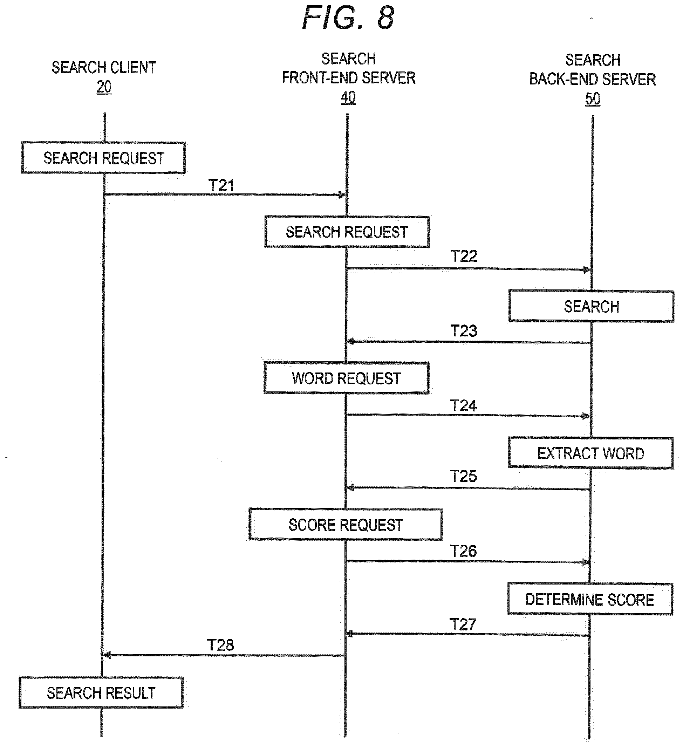

Next, a flow of an example of processing executed in the present embodiment will be described with reference to sequence diagrams of FIGS. 7 and 8.

FIG. 7 is a sequence diagram of processing of registering a set of seed documents.

The user uses the seed document setting screen 611 provided by the search request input unit 310 included in the seed document setting client 30 to input the search input for setting the seed document. The search request including the search input is transmitted from the search request input unit 310 of the seed document setting client 30 to the search front-end server 40 (T11).

The search request unit 410 of the search front-end server 40 receives the search request, and transmits the search request to the search back-end server 50 (T12).

In response to the search request, the search unit 510 of the search back-end server 50 searches for a document from the document database 560 using the search index 550 (specifically, for example, searches for a document matching the search input (for example, one or more keywords) included in the search request), and returns a search result thereof (for example, a document set including one or more found documents) to the search front-end server 40 (T13).

The topic word request unit 420 of the search front-end server 40 transmits an extraction request of the topic word to the search back-end server 50 in order to extract the topic word from the obtained search results (T14) . The document set as the obtained search result is associated with the extraction request.

In response to the extraction request, the topic word extraction unit 520 of the search back-end server 50 extracts the topic word set using the search index 550 from the document set associated with the extraction request, and returns a topic word set to the search front-end server 40 (T15). The topic word set includes one or more topic words and a weight of each of the one or more topic words.

The document score determination request unit 430 of the search front-end server 40 transmits, for the document set (search result) returned in T13, a score determination request for determining the document score using the topic word set returned in T15 to the search back-end server 50 (T16). The document set returned in T13 and the topic word set returned in T15 are associated with the score determination request.

In response to the score determination request, the document score determination unit 530 of the search back-end server 50 determines the document score using the topic word set associated with the request for each document constituting the document set associated with the request, and returns a score determination result (document score for each document) thereof to the search front-end server 40 (T17).

The seed document registration request unit 440 of the search front-end server 40 selects, based on a predetermined seed document standard (for example, a standard of "documents of document score top 200"), a set of seed documents (one or more seed documents) from the document set returned in T13 based on the score determination result returned in T17, and transmits a setting request for setting the selected set of seed documents to the search back-end server 50 (T18). The selected set of seed documents is associated with the setting request. The "seed document standard" is a condition related to a document corresponding to a seed document. Further, the "seed document" is a document having relatively high potential of being a useful document, specifically, for example, a document having a relatively high document score in the document set matching the search input (in other words, a search condition) input in the seed document registration processing shown in FIG. 7. Therefore, the "document score" is a score indicating a potential of being a useful document. The "useful document" is a document which is useful to the user, for example, a document which contributes to solution of a research problem of the user himself. A specific example of the useful document will be described later.

In response to the setting request, the seed document registration unit 540 of the search back-end server 50 registers the set of seed documents associated with the request in the seed document database 570.

The above is an example of the registration of the set of seed documents. The set of seed documents may include another document in place of or in addition to one or more document set found in the document database 560. For example, one or more seed documents may be selected from a store different from the document database 560, and a set of seed documents including the one or more selected seed document may be registered as the seed document database 570. Therefore, the seed document setting client 30 may not be provided.

FIG. 8 is a sequence diagram of processing of searching for a useful document.

The user inputs a search input for searching a useful document from the document database 560 by using the search request input screen 411 provided by the search request input unit 210 included in the search client 20. A search request including the input search input is transmitted from the search request input unit 210 of the search client 20 to the search front-end server 40 (T21).

The search request unit 410 of the search front-end server 40 receives the search request, and transmits the search request to the search back-end server 50 (T22).

In response to the search request, the search unit 510 of the search back-end server 50 searches for a document from the document database 560 using the search index 550 (specifically, for example, searches for a document matching the search input (for example, one or more keywords) included in the search request), and returns a search result thereof (for example, a document set including one or more found documents) to the search front-end server 40 (T23).

The topic word request unit 420 of the search front-end server 40 transmits an extraction request of the topic word to the search back-end server 50 in order to extract the topic word from the set of seed documents included in the seed document database (T24).

In response to the extraction request, the topic word extraction unit 520 of the search back-end server 50 extracts the topic word set from the seed document database 570 using the search index 550, and returns the topic word set to the search front-end server 40 (T25). The topic word set is an example of the useful document model including the one or more topic words and the weight of each of the one or more topic words.

The document score determination request unit 430 of the search front-end server 40 transmits, for the document set (search result) returned in T23, a score determination request for determining the document score using the topic word set returned in T25 to the search back-end server 50 (T26). The document set returned in T23 and the topic word set returned in T25 are associated with the score determination request.

In response to the score determination request, the document score determination unit 530 of the search back-end server 50 determines the document score using the topic word set associated with the request for each document constituting the document set associated with the request, and returns a score determination result (document score for each document) thereof to the search front-end server 40 (T27). The score determination result is returned to the search client 20 as it is by the search front-end server 40 (T28), and is displayed on the document list screen 511 of the search result screen 580 by the search result display unit 220 of the search client 20. A document having a higher document score is more likely to be a useful document.

Next, a case of searching for a metabolic reaction related to material production will be described as an example. In a material production field, there is a request to find a useful document (typically a document describing past cases for a research problem of the user himself) for a designed metabolic pathway. Specifically, for example, there is a request to search a predetermined document database (for example, a database called "PubMed") for past cases related to at least one of introduction, enhancement and suppression of a reaction. In this case, the "useful document" is a document describing cases which contribute to production of a compound serving as a target compound (substances to be produced), for example, a document describing a case in which a reaction specified with a search input contributes to the material production (for example, a document describing "successful production of a target compound T1 by introduction of gene GI", "increase in production of a target compound T2 by deletion of gene G2", and the like).

In the present embodiment, the set of seed documents is set so that the score can be determined by using such documents as the useful documents. When an example is described with reference to the sequence diagram of FIG. 7, a search input (for example, keywords such as "Metabolic Engineering" and/or "Microbial Cell Factories") for searching for a document set published in journal articles related to the material production is input to the search request input unit 310 of the seed document setting client 30.

The journal articles related to the material production do not necessarily include only journals related to the material production. Therefore, with the processing procedure of the sequence diagram of FIG. 7, a document score indicating a degree of being related to the material production can be determined for each document constituting the document set published in the journal articles related to the material production (T14 to T17). The set of seed documents obtained by selecting a part of the documents from this document set based on a certain standard (for example, documents of document score top 200) is a document set having a high usefulness in which a document set having a low usefulness is excluded from the document set found according to the search request including the search input by the user (T17 and T18).

Next, a search example related to the reaction will be described with reference to the sequence diagram of FIG. 8. Here, an example of searching for a gene expressing an enzyme related to the reaction will be described.

In order to search for the reaction of enzyme No. 2.2.1.6, the user searches for a gene expressing an enzyme of enzyme No. 2.2.1.6 using a predetermined database. As a result, alsS, brnP, budB, ilvB, ilvB1, ilvB2, ilvG, ilvH, ilvI, ilvK, ilvM, ilvN, ilvX, and ilvY are obtained as genes. A document search (for example, an OR search) is executed from the document database 560 according to a search request including a search input which includes these gene names (T22 and T23).

A topic word set is extracted from the document set (that is, a set of seed documents) having the high usefulness created based on the above-described journal articles related to the material production, and a document score is determined using the extracted topic word set, for each document constituting the document set as a result obtained by searching by the gene names (T24 to 27).

As a result, among the document set including at least one of the above-described gene names, a title and a document score of a document (material production document) having high degree of being related to the material production are displayed on the search client 20 as a search result.

The search is executed with the gene name in the above-described example. However, when the search is executed with a name of a substrate of a reaction, a name or a number of an enzyme (for example, at least a part of the numbers (for example, upper x digits (x is a natural number)), a name of a product, or a combination thereof, a document having a high potential of being a material production document (useful document) can be presented by the same procedure.

The above-described first embodiment can be summarized, for example, as follows.

The document search system includes the topic word extraction unit 520, the search unit 510, and the document score determination unit 530. The topic word extraction unit 520 extracts one or more topic words from the seed document database 570 (an example of a set of seed documents), and creates a topic word set (an example of a useful document model) including the one or more topic words and the weight of each of the one or more topic words. The search unit 510 extracts a document set (one or more documents) matching a search condition from the document database 560 (an example of a document search range including one or more documents) according to a search request in which the search condition is specified. The document score determination unit 530 determines, for each of the one or more documents in the extracted document set, the document score of the document based on the topic word set, and outputs (for example, displays) the search result on descending order of the document score. The document score of each document found by the search unit 510 is a score determined based on the topic word set including the topic words extracted from the set of seed documents which may be the useful documents and weights thereof. Therefore, the document score refers to the potential of the document being a useful document. Since the search result on descending order of the document score is displayed, the user can efficiently find a useful document for the user.

The document search system may further include the seed document registration unit 540 which registers the set of seed documents in the seed document database 570. The search unit 510 may search the document database 560 for the one or more documents according to another search request which is a search request including a search condition input for registering the set of seed documents prior to the search request. The topic word extraction unit 520 may extract the one or more topic words from the one or more documents and determine the weight of each of the one or more topic words. Further, the document score determination unit 530 may determine, for each of the one or more documents, the document score based on the one or more topic words and the weight of each topic word. The seed document registration unit may register a set of documents having relatively high determined document scores among the one or more searched documents according to another search request to the seed document database 570 as the set of seed documents. In this way, the documents constituting the set of seed documents are documents obtained from the document database 560 referenced in the search for the useful document. In other words, a source of the documents constituting the set of seed documents is the same as the document search range of the useful documents. Therefore, a high accuracy of the document score (document score determined for the document searched in the useful document search) based on the topic word set generated from the set of seed documents is expected.

The useful document is a document in which cases which contribute to the production of the compound serving as the target compound are described. The search condition relates to a metabolic pathway designed to produce the target compound and includes at least one of a compound name of the target compound, a reaction name of at least one reaction among one or more reactions constituting the metabolic pathway, a metabolite name of one or more metabolites constituting the metabolic pathway, at least a part of enzyme numbers, an enzyme name, and one or more gene names. Accordingly, for the metabolic pathway designed by the user, the user can efficiently find a document in which a past case is described.

Once the created topic word set (an example of the useful document model) is saved, a useful document search may be performed in subsequent search requests without creating the topic word set. However, each time the topic word extraction unit 520 receives the search request, the topic word extraction unit 520 may respond to the search request and create the topic word set based on the set of seed documents. Accordingly, it is expected that the useful document search will always be based on the latest set of seed documents. For example, when the number of documents registered in the document database 560 increases frequently, the number of documents included in the set of seed documents may increase frequently. In such a case, it is considered effective to create the topic word set based on the set of seed documents each time the search request is received (each time the useful document search is executed) .

In the generation of the topic word set described above, the tf * idf method can be used. Specifically, for example, processing is as follows. That is, according to a term frequency (TF), a word appearing a lot in a document has high importance, and a word appearing disproportionately has high importance. According to an inversed document frequency (IDF), a word appearing in many documents has low importance. For each word, the number of documents in which the word appears is a document frequency (DF), and a reciprocal of DF is IDF. The word appearing in many documents has a small IDF, and a word appearing in only a small number of documents has a large IDF. A weight (q, t) which is a weight of a word t in a document set q can be calculated using an importance calculation formula as Equation 1.

$$weight(q,t) = \log\left(1 + \frac{Nr(w)}{DF(.|t)}\right)\left(\frac{1}{DF(.|q)}\right) \sum_{d\ in\ q} \frac{1 + \log\big(TF(t|d)\big)}{1 + \log\left(\frac{TF(.|d)}{DF(.|d)}\right)}$$

q means the set of seed documents. TF(t|d) is a frequency of t in a document d. TF(. |D) is the number of words in d. DF(. |D) means the number of different words in d. In other words, inside $\Sigma$ is an index of how much appearance of t is shifted from an average frequency of the word in d, which corresponds to TF. This is calculated for all seed documents and divided by the number of seed documents DF(. |q) to calculate an average. Nr(w) means the total number of documents. DF(. |t) means DF of t. log(1+Nr(w)/DF(. |t)) means IDF.

[Second Embodiment]

A second embodiment will be described. At this time, differences from the first embodiment will be mainly described, and common points with the first embodiment will be omitted or simplified.

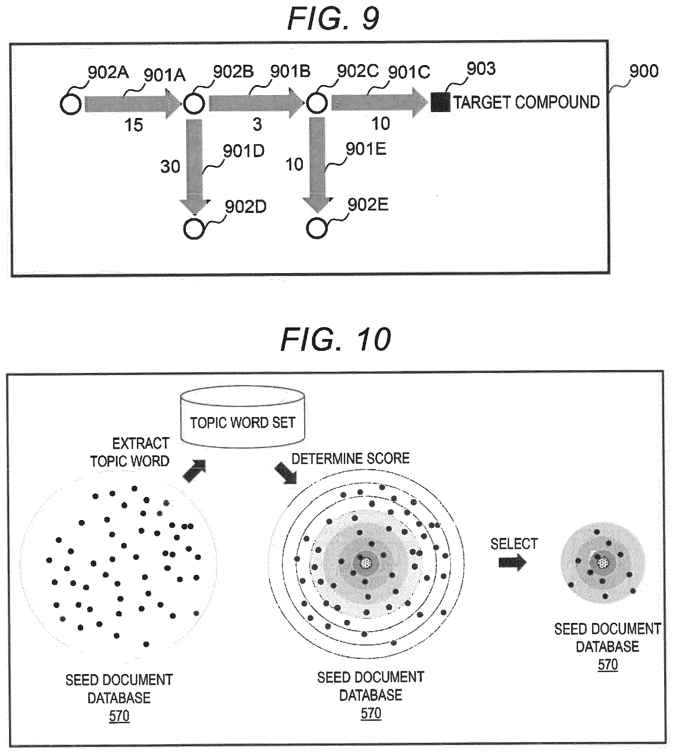

FIG. 9 shows an outline of the second embodiment.

According to the first embodiment, the document database 560 can be searched for documents according to the search request including the search input including the information on the reaction, and the document set of the obtained documents can be presented in descending order of the document score.

A fact that there are many documents having a high document score in a document set containing a certain gene suggests that the gene (reaction) is often used for the material production. Therefore, by setting a threshold in the document score and counting the number of documents above the threshold, a material production degree of the reaction can be inferred.

Therefore, in the second embodiment, the document score determination unit 530 displays a search result screen 900 shown in FIG. 9 in addition to the search result screen 580 shown in FIG. 5 through the search result display unit 220 of the search client 20. In FIG. 9, a metabolite object 902 represents a metabolite (substrate or product), a reaction object 901 represents a reaction, and a target compound object 903 represents a target compound. Specifically, the search result screen 900 relates to the designed metabolic pathway and includes a plurality of reaction objects 901A to 901E corresponding to a plurality of reactions constituting the metabolic pathway, metabolite objects 902A to 902E corresponding to metabolites before or after the reaction, and the target compound object 903. The reaction object 901 is a display object (for example, a graphic) representing the reaction. For example, each of the reaction objects 901A to 901C means enhancement of the reaction, and each of the reaction objects 901D and 901E means suppression of the reaction. The metabolite object 902 is a display object representing the metabolite. The target compound object 903 is a display object representing the target compound.

In the search result screen 900, for each of the one or more reactions constituting the designed metabolic pathway, the number of documents is displayed which is a value associated with a reaction object of the reaction and a value representing the number of documents whose document score is equal to or higher than a threshold for the reaction. By looking at the number of documents for each reaction, the user can infer the material production degree of the reaction, for example, a reaction corresponding to the reaction object 901D associated with the number of documents "30" is likely to be a reaction which is often manipulated in the material production, and a reaction corresponding to the reaction object 901B associated with the number of documents "3" is likely to be a reaction which is not often manipulated in the material production.

In this way, in the second embodiment, the document score determination unit 530 outputs, for each of the one or more reactions constituting the designed metabolic pathway, the number of documents which is a value associated with the reaction object 901 representing the reaction and a value representing the number of documents whose document score is equal to or higher than the threshold for the reaction. Accordingly, as described above, the user can infer the material production degree of the reaction by looking at the number of documents for each reaction.

When the user specifies (for example, clicks) the reaction object 901 (or the number of documents associated with the reaction) of a reaction desired by the user, the document score determination unit 530 may display the reaction on the search result screen 580 shown in FIG. 5. That is, the search result presented by the search result screen 580 is a search result on descending order of the document score of the documents extracted for the reaction desired by the user. In this way, the user can efficiently find useful documents for the desired reaction.

[Third Embodiment]

A third embodiment will be described. At this time, differences from the first and second embodiments will be mainly described, and common points with the first and second embodiments will be omitted or simplified.

FIG. 10 shows an outline of the third embodiment.

It is considered that the larger the number of seed documents constituting a set of seed documents is, the higher a search accuracy of useful document search (for example, an accuracy of a document score determined for a searched document) is.

However, when a topic word set (an example of a useful document model) is created from the set of seed documents each time a useful document search is executed, it is considered that a search speed of the useful document search is slower as the number of seed documents increases. This is because it takes time to create the topic word set.

Therefore, in the third embodiment, the number of seed documents can be reduced while reducing a decrease in the search accuracy of the useful document search.

Specifically, as shown in FIG. 10, the document score determination unit 530 determines, based on a topic word set created from a set of seed documents, a document score for each of one or more seed documents in the set of seed documents in the seed document database 570. Then, the document score determination unit 530 updates the set of seed documents by narrowing down the set of seed document to a seed document whose determined document score is equal to or higher than a threshold (for example, by narrowing down to seed documents whose document scores are top x (x is a natural number) , and replaces the set of seed documents in the seed document database 570 with the updated set of seed documents. Accordingly, documents which are unlikely to be the useful documents are excluded from the set of seed documents, and the number of documents constituting the updated set of seed documents is smaller than the number of documents constituting the set of seed documents before the update. Since the topic word set is created in the useful document search from the set of seed documents after such update, the number of seed documents can be reduced while reducing a decrease in the search accuracy of the useful document search.

[Fourth Embodiment]

A fourth embodiment will be described. At this time, differences from the first to third embodiments will be mainly described, and common points with the first to third embodiments will be omitted or simplified.

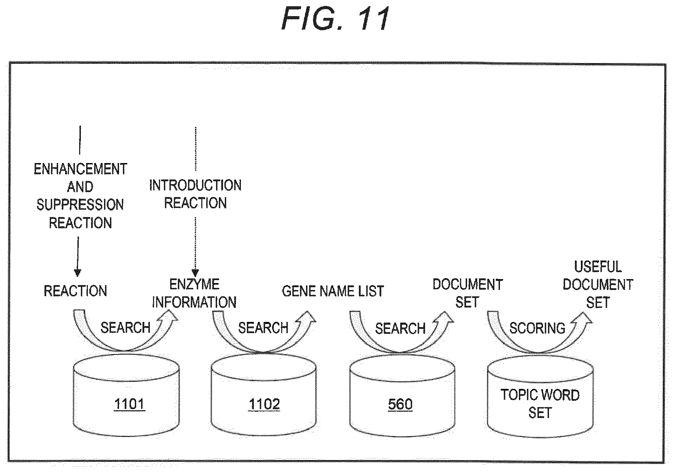

FIG. 11 shows an outline of the fourth embodiment.

A document search system can execute a useful document search referencing a plurality of databases step by step in response to a search request including a search condition including a reaction name. Specifically, for example, as shown in FIG. 11, the search unit 510 identifies enzyme information (for example, at least a part of enzyme numbers or an enzyme name) from a first database 1101 (an example of a first information set) based on the reaction name included in the search condition, identifies a gene name list (one or more gene names) from a second database 1102 (an example of a second information set) based on the identified enzyme information, and extracts a document set (one or more documents) from the document database 560 based on the specified gene name. Then, the document score determination unit. 530 determines the document score for each document constituting the document set using the topic word set created from the seed document database 570. In this way, the enzyme information is identified using the reaction name included in the search condition as a key, the gene name list is identified based on the enzyme information, and the document can be automatically searched for using the gene name list.

The search condition may include the enzyme information corresponding to the reaction name instead of the reaction name. In this case, the search unit 510 may identify the gene name list from the database 1102 (an example of a predetermined information set) based on the enzyme information in the search condition, and may extract the document set from the document database 560 based on the specified gene name list. In this case, the gene name list can be identified using the enzyme information included in the search conditions as a key, and the document can be automatically searched for using the gene name list.

Although some embodiments are described above, the embodiments are examples for describing the invention and are not intended to limit the scope of the invention to the embodiments. The invention can be implemented in various other forms.

**Claims**

1. A document search system comprising:

   a topic word extraction unit (520) configured to extract one or more topic words from a set of seed documents of one or more seed documents and create a useful document model which is a model including the one or more topic words and a weight (q, t) of each of the one or more topic words, a seed document being a document which is a useful document;
   a search unit (510) configured to extract one or more documents matching a search condition from a document search range including one or more documents according to a search request in which the search condition is specified; and
   a document score determination unit (530) configured to determine, for each of the one or more extracted documents, a document score of the document based on the useful document model and output a search result on descending order of document scores of the one or more extracted documents.

2. The document search system according to claim 1, further comprising:

   a seed document registration unit (540) configured to register the set of seed documents, wherein
   the search unit (510) searches the document search range for one or more documents according to another search request which is a search request including a search condition input for registering the set of seed documents prior to the search request,
   the topic word extraction unit (520) extracts one or more topic words from the one or more documents and determines a weight (q, t) of each of the one or more topic words,
   the document score determination unit (530) determines, for each of the one or more documents, a document score based on the one or more topic words and the weight (q, t) of each topic word, and
   the seed document registration unit (540) registers a set of documents having relatively high determined document scores among the searched one or more documents according to the other search request as the set of seed documents.

3. The document search system according to claim 1, wherein
   the useful document is a document in which cases which contribute to production of a compound serving as a target compound (T1, T2) are described, and
   the search condition relates to a metabolic pathway designed to produce the target compound (T1, T2), and includes at least one of a compound name of the target compound (T1, T2), a reaction name of at least one reaction among one or more reactions constituting the metabolic pathway, a metabolite name of one or more metabolites constituting the metabolic pathway, at least a part of enzyme numbers, an enzyme name, and one or more gene names (T22, T23, T24, T25, T26, T27).

4. The document search system according to claim 1, wherein
   each time the topic word extraction unit (520) receives a search request, the topic word extraction unit (520) responds to the search request and creates the useful document model based on the set of seed documents.

5. The document search system according to claim 3, wherein
   the document score determination unit (530) outputs, for each of the one or more reactions constituting the designed metabolic pathway, the number of documents which is a value associated with a display object representing the reaction and is a value representing the number of documents whose document score is equal to or higher than a threshold for the reaction.

6. The document search system according to claim 5, wherein
   regarding a specified reaction among the one or more reactions, the output search result is a search result on descending order of document scores of documents extracted for the reaction.

**7.** The document search system according to claim 4, wherein
the document score determination unit (530) determines, based on the useful document model, the document score for each of the one or more seed documents in the set of seed documents, and updates the set of seed documents by narrowing down the set of seed documents to a seed document whose determined document score is equal to or higher than a threshold.

**8.** The document search system according to claim 3, wherein
the search unit (510) is configured to:

identify enzyme information which is at least a part of the enzyme numbers or the enzyme name from a first information set based on a reaction name included in the search condition including the reaction name,
identify, based on the identified enzyme information, a gene name list including one or more gene names (T22, T23, T24, T25, T26, T27) from a second information set, and
extract the one or more documents from the document search range based on the identified gene name list.

**9.** The document search system according to claim 3, wherein
the search unit (510) is configured to:

identify, based on enzyme information included in the search condition including the enzyme information which is at least a part of the enzyme numbers or the enzyme name, a gene name list including one or more gene names (T22, T23, T24, T25, T26, T27) from a predetermined information set, and
extract the one or more documents from the document search range based on the identified gene name list.

**10.** A document search method comprising:

extracting, by a computer, one or more topic words from a set of seed documents of one or more seed documents, a seed document being a document which is a useful document;
creating, by a computer, a useful document model which is a model including the one or more topic words and a weight $(q, t)$ of each of the one or more topic words;
extracting, by a computer, one or more documents matching a search condition from a document search range including one or more documents according to a search request in which the search condition is specified;
determining, by a computer, for each of the one or more extracted documents, a document score of the document based on the useful document model; and
outputting, by a computer, a search result on descending order of document scores of the one or more extracted documents .

**11.** A computer program configured to cause a computer to:

extract one or more topic words from a set of seed documents of one or more seed documents, a seed document being a document which is a useful document;
create a useful document model which is a model including the one or more topic words and a weight $(q, t)$ of each of the one or more topic words;
extract one or more documents matching a search condition from a document search range including one or more documents according to a search request in which the search condition is specified;
determine for each of the one or more extracted documents, a document score of the document based on the useful document model; and
output a search result on descending order of document scores of the one or more extracted documents.

## FIG. 1

## FIG. 2

520

TOPIC WORD EXTRACTION UNIT

521

WORD FREQUENCY
ACQUISITION UNIT

522

IMPORTANCE
CALCULATION UNIT

## FIG. 3

530

DOCUMENT SCORE
DETERMINATION UNIT

531

WORD FREQUENCY
ACQUISITION UNIT

532

SCORE CALCULATION
UNIT

## FIG. 4

411

| | Search |

211                                                                    212

## FIG. 5

580          411

| | Search |

| RANKING | DOCUMENT SCORE | TITLE |
|---------|----------------|-------|
| 1 | SCORE 1 | DOCUMENT TITLE 1 |
| 2 | SCORE 2 | DOCUMENT TITLE 2 |
| 3 | SCORE 3 | DOCUMENT TITLE 3 |
| 4 | SCORE 4 | DOCUMENT TITLE 4 |
| 5 | SCORE 5 | DOCUMENT TITLE 5 |

511

## FIG. 6

611

| | Set |

311                                                                    312

# FIG. 7

| SEED DOCUMENT<br>SETTING CLIENT<br>30 | SEARCH<br>FRONT-END SERVER<br>40 | SEARCH<br>BACK-END SERVER<br>50 |
|---|---|---|

SEARCH REQUEST

T11

SEARCH REQUEST

T12

SEARCH

T13

WORD REQUEST

T14

EXTRACT WORD

T15

SCORE REQUEST

T16

DETERMINE SCORE

T17

REGISTRATION
REQUEST

T18

REGISTER

# FIG. 8

| SEARCH CLIENT 20 | SEARCH FRONT-END SERVER 40 | SEARCH BACK-END SERVER 50 |

SEARCH REQUEST

T21

SEARCH REQUEST

T22

SEARCH

T23

WORD REQUEST

T24

EXTRACT WORD

T25

SCORE REQUEST

T26

DETERMINE SCORE

T27

T28

SEARCH RESULT

# FIG. 9

# FIG. 10

# FIG. 11

ENHANCEMENT AND SUPPRESSION REACTION

INTRODUCTION REACTION

REACTION — SEARCH — ENZYME INFORMATION — SEARCH — GENE NAME LIST — SEARCH — DOCUMENT SET — SCORING — USEFUL DOCUMENT SET

1101    1102    560    TOPIC WORD SET

**EUROPEAN SEARCH REPORT**

Application Number

EP 21 15 2950

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/230031 A1 (IKEDA TETSUYA [JP] ET AL) 12 October 2006 (2006-10-12) <br> * paragraph [0018] * <br> ----- | 1-11 | INV. <br> G06F16/335 <br> G06F40/00 <br> G16B45/00 |

TECHNICAL FIELDS SEARCHED (IPC)

G06F
G16B

The present search report has been drawn up for all claims

| Place of search | Date of completion of the search | Examiner |
|---|---|---|
| The Hague | 31 May 2021 | Rameseder, Jonathan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 15 2950

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2021

| Patent document cited in search report | Publication date | Patent family member(s) | Publication date |
|---|---|---|---|
| US 2006230031 A1 | 12-10-2006 | JP 4825544 B2<br>JP 2007149047 A<br>US 2006230031 A1 | 30-11-2011<br>14-06-2007<br>12-10-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000155758 A